# NEW EUROPEAN PATENT SPECIFICATION

(11) **EP 3 581 244 B2**
(45) Date of publication and mention of the opposition decision: **03.01.2024**
(45) Mention of the grant of the patent: 31.03.2021
(21) Application number: 19180936.7
(22) Date of filing: 21.10.2011
(51) Int. Cl.: A61N 7/00, A61B 5/00

(54) **MEDICAL DEVICE**
MEDIZINPRODUKT
DISPOSITIF MÉDICAL

(30) Priority: 22.10.2010 US 40575710 P; 06.05.2011 US 201161483445 P
(43) Date of publication of application: 18.12.2019
(62) Divisional of application: 11779297.8
(73) Proprietor: Smith & Nephew, Inc., Memphis, TN 38116 (US)
(72) Inventor: TANIS, Kevin Jay, Arlington, TN 38002 (US); ZHANG, Jin, Memphis, TN 38117 (US)
(74) Representative: Smith & Nephew

(56) References cited:
- EP-B1- 1 566 201
- WO-A2-2009/137699
- WO-A2-2009/151645
- US-A- 5 634 939
- US-A1- 2002 013 516
- US-A1- 2008 097 793
- US-A1- 2010 022 990

## Description

### FIELD

This description relates to a medical device.

### BACKGROUND

Medical devices can provide treatment for a variety of health conditions. In some instances, a patient has a degree of control over treatment with a medical device. For example, a patient may be able to initiate treatment with a medical device. The capabilities of a medical device determine to a large degree the way that the patient and others interact with the medical device. In particular, it is important that a medical device be capable of providing effective treatment and a positive patient experience. Examples of communication systems between a remote computer system and a medical device are given in WO 2009/137699 for example.

### SUMMARY

The invention is defined in the independent claim. Preferred features are set out in the dependent claims.

In one aspect, a medical device includes a treatment module configured to apply a medical treatment and one or more storage devices storing a device identifier that identifies the medical device. The medical device includes one or more one or more processing devices are configured to control the treatment module to apply a medical treatment, collect compliance information that reflects a patient's compliance with a treatment regimen involving the treatment module, access the device identifier from the one or more storage devices, and send the collected compliance information with the accessed device identifier to a server system. The server system is configured to receive the compliance information and the device identifier, and to determine a patient identifier based on the device identifier. The server system is configured to store the compliance information in association with the determined patient identifier, and provide access to the compliance information to one or more users in response to the one or more users submitting an inquiry associated with the patient identifier to the server system.

Implementations may include one or more of the following. For example, the treatment module includes at least one ultrasound transducer and at least one driver circuit coupled to the ultrasound transducer, and to control the treatment module to apply a medical treatment, the one or more processing devices are configured to control the driver circuit such that the driver circuit causes the at least one ultrasound transducer to produce ultrasound with therapeutic properties. The compliance information includes at least one of: a number of treatments provided by the medical device, a date or time that a treatment was provided by the medical device, and a duration that a treatment was provided by the medical device. The one or more processing devices being configured to collect compliance information includes being configured to record information about use of the medical device on the one or more storage devices. The one or more processing devices being configured to collect compliance information includes being configured to (i) identify a treatment regimen that identifies a prescribed use of the medical device, (ii) compare the information about the recorded use to the prescribed use of the medical device, and (iii) generate information indicating a degree that the recorded use matches the prescribed use. The medical device includes a wireless communication module. The wireless communication module includes a cellular transceiver. To send the collected compliance information, the one or more processing devices are configured to send the collected compliance information to the server system over a cellular network using the cellular transceiver. The one or more processing devices are configured to send the collected compliance information with the accessed device identifier after the patient has used the medical device a predefined number of times. The one or more processing devices are further configured to detect one or more errors of the medical device and send information about the detected errors with the accessed device identifier to a server system that is configured to receive the information about the errors and the device identifier. The one or more processing devices are further configured to receive service information to address the detected errors. The one or more processing devices are further configured to receive one or more messages to display on a user interface of the medical device. The server system is further configured to provide access to the stored compliance information for the plurality of patients. The one or more users include one or more of a representative of an insurance provider of the patient, a physician of the patient, and a caregiver for the patient. Access to the compliance information is limited based on the relationship of the user to the patient.

In another general aspect, a server system includes one or more processing devices, and one or more storage devices storing instructions that, when executed by the one or more processing devices, cause the one or more processing devices to: receive compliance information that reflects a patient's compliance with a treatment regimen involving treatment with a medical device; receive a device identifier that identifies the medical device; determine a patient identifier based on the device identifier; store the compliance information in association with the determined patient identifier; and provide access to the compliance information to one or more users in response to the one or more users submitting an inquiry associated with the patient identifier to the server system.

Implementations may include one or more of the following features. For example, to receive compliance information, the server system is configured to receive compliance information that reflects a patient's compliance with a treatment regimen involving ultrasound with therapeutic properties produced by the medical device. The instructions further cause the one or more processing devices to receive compliance information for each of a plurality of patients, where each of the plurality of patients is associated at least one of a plurality of medical devices, and store the compliance information for each of the plurality of patients. The instructions further cause the one or more processing devices to provide access to the stored compliance information for the plurality of patients to each of a plurality of users. The compliance information includes at least one of a number of treatments provided by the medical device, a date or time that a treatment was provided by the medical device, and a duration that a treatment was provided by the medical device. The one or more users include one or more of a representative of an insurance provider of the patient, a physician of the patient, and a caregiver for the patient. Access to the compliance information is limited based on a relationship of the user to the patient. The instructions further cause the one or more processing devices to receive information identifying one or more errors of the medical device and transmit service information to address the detected errors of the medical device. The instructions further cause the one or more processing devices to transmit one or more messages for the patient to the medical device.

In another general aspect, a system includes a medical device including one or more processing devices configured to control a treatment module of the medical device to (i) apply a medical treatment, (ii) collect compliance information that reflects a patient's compliance with a treatment regimen involving the medical treatment applied by the treatment module, and (iii) send the collected compliance information with a device identifier. The system includes a server system that includes one or more processing devices configured to receive the compliance information from the medical device, and provide access to the compliance information to one or more users in response to the one or more users submitting an inquiry associated with the patient identifier to the server system.

Implementations may include one or more of the following features. For example, to control the treatment module of the medical device, the one or more processing devices of the medical device are configured to control a driver circuit coupled to an ultrasound transducer to produce ultrasound with therapeutic properties. To collect compliance information, the one or more processing devices of the medical device are configured to collect compliance information that reflects the patient's compliance with a treatment regimen involving the produced ultrasound. The one or more processing devices of the server system are configured to receive compliance information for each of a plurality of patients, where each of the plurality of patients is associated at least one of a plurality of medical devices, and store the compliance information for each of the plurality of patients. The one or more processing devices of the server system are configured to provide access to the stored compliance information for the plurality of patients to each of a plurality of users. The compliance information includes at least one of a number of treatments provided by the medical device, a date or time that a treatment was provided by the medical device, and a duration that a treatment was provided by the medical device. The one or more processing devices of the medical device being configured to collect compliance information includes being configured to record information about use of the medical device on one or more storage devices of the medical device. The one or more processing devices of the medical device being configured to collect compliance information includes being configured to (i) identify a treatment regimen that identifies a prescribed use of the medical device, (ii) compare the information about the recorded use to the prescribed use of the medical device, and (iii) generate information indicating the degree that the recorded use matches the prescribed use. The one or more users include one or more of a representative of an insurance provider of the patient, a physician of the patient, and a caregiver for the patient. Access to the compliance information is limited based on a relationship of the user to the patient. The one or more processing devices of the medical device are further configured to detect one or more errors of the medical device and send information about the detected errors with the accessed device identifier to the server system. The one or more processing devices of the server system are further configured to transmit service information to address the detected errors to the medical device. The medical device includes a wireless communication module. The wireless communication module includes a cellular transceiver. The one or more processing devices of the medical device are configured to send the collected compliance information to the server system over a cellular network using the cellular transceiver. The one or more processing devices of the medical device are configured to send the collected compliance information with the accessed device identifier after the patient has used the medical device a predefined number of times.

In another general aspect, a method includes receiving, by a server system, compliance information that reflects a patient's compliance with a treatment regimen involving treatment with a medical device. The method includes receiving a device identifier that identifies the medical device, determining a patient identifier based on the device identifier, storing the compliance information in association with the determined patient identifier, and providing access to the compliance information to one or more users in response to the one or more users submitting an inquiry associated with the patient identifier to the server system.

Implementations may include one or more of the following features. For example, receiving compliance information for each of a plurality of patients, each of the plurality of patients being associated at least one of a plurality of medical devices, and storing the compliance information for each of the plurality of patients. The method includes providing access to the stored compliance information for the plurality of patients to each of a plurality of users. The compliance information includes one or more of a number of treatments provided by the medical device, a date or time that a treatment was provided by the medical device, and a duration that a treatment was provided by the medical device. The one or more users include one or more of a representative of an insurance provider of the patient, a physician of the patient, and a caregiver for the patient. The method includes limiting access to the compliance information based on a relationship of the one or more users to the patient. The method includes receiving information identifying one or more errors of the medical device and transmitting service information to address the detected errors of the medical device. The method includes transmitting one or more messages for the patient to the medical device.

In another general aspect, an enabling device includes a communication module operable to transmit activation data to a medical device, the activation data being capable of activating the medical device, The enabling device includes one or more data storage devices storing authorization information indicating a number of authorized activations of medical devices that the enabling device is authorized to perform. The enabling device includes one or more processing devices configured to receive user input indicating that a medical device should be activated and determine, based on the authorization information, whether activation of the medical device is authorized. The one or more processing devices are configured to, if the determination indicates that the activation of the medical device is authorized, control the communication module to transmit the activation data, and update the authorization information to decrease the number of authorized activations remaining for the enabling device. The one or more processing devices are configured to, if the determination indicates that the treatment is not authorized, control the treatment module such that the activation data are not transmitted.

Implementations may include one or more of the following features. For example, the communication module is a wireless communication module configured to wirelessly transmit the activation data to the medical device. The enabling device is associated with an inventory of medical devices, and the number of authorized activations of medical devices is based on the associated inventory. The one or more storage devices store an identifier for a user of the enabling device, and the identifier for the user is associated with the inventory of medical devices. The one or more processing devices are configured to receive an activation confirmation message indicating that activation was successful. The one or more processing devices are configured to update the authorization information to decrease the number of authorized activations remaining for the enabling device in response to receiving an activation confirmation message. The communication module is further configured to receive, over a network, second authorization information indicating a change in the number of authorized activations that the enabling device is authorized to perform. The one or more processing devices are further configured to update the information indicating the number of authorized activations according to the change in the number of authorized activations indicated by the second authorization information. The communication module is further configured to receive, over a network, second authorization information indicating an additional number of authorized activations the enabling device is authorized to perform. The one or more processing devices are further configured to update the information indicating the number of authorized activations to increase the number of authorized activations by the additional number indicated by the second authorization information.

In another general aspect, a method performed by one or more processing devices includes receiving user input indicating that a medical device should be activated, accessing authorization information indicating a number of authorized activations, and determining, based on the authorization information, that activation of the medical device is authorized. The method includes, in response to determining that the activation of the medical device is authorized, (i) transmitting activation data to the medical device to activate the medical device, and (ii) updating the authorization information to decrease the number of authorized activations remaining.

Implementations may include one or more of the following features. For example, the method includes wirelessly transmitting the activation data to the medical device. Accessing the authorization information includes accessing authorization information that indicates a number of authorized activations of medical devices that can be performed, the number being based on an inventory associated with an enabling device or a user of the enabling device. The method includes storing an identifier for a user of an enabling device, and the identifier for the user is associated with the inventory of medical devices. The method includes receiving an activation confirmation message indicating that activation was successful. The method includes decreasing the number of authorized activations remaining in response to receiving the activation confirmation message. The method includes receiving, over a network, second authorization information indicating a change in the number of authorized activations, and in response to receiving the second authorization information, updating the information indicating the number of authorized activations according to the change in the number of authorized activations indicated by the second authorization information. The method includes receiving, over a network, second authorization information indicating an additional number of authorized activations, and in response to receiving the second authorization information, updating the information indicating the number of authorized activations to increase the number of authorized activations by the additional number indicated by the second authorization information.

In another general aspect, a system includes a medical device including a treatment module operable to apply a treatment to a patient. The medical device is configured to disallow treatment using the treatment module until activated by an enabling device. The system includes an enabling device operable to activate the medical device. The enabling device is configured to activate up to a particular number of medical devices, and to not activate more than the particular number of medical devices. The system includes a server system configured to monitor an inventory of medical devices associated with the enabling device and set the particular number of medical devices that the enabling device can activate based on the number of medical devices in the inventory.

Implementations may include one or more of the following features. For example, the treatment module includes at least one ultrasound transducer and at least one driver circuit coupled to the ultrasound transducer. The enabling device is configured to activate the medical device by transmitting activation data over a wireless communication link between the enabling device and the medical device. The medical device is configured to allow treatments using the treatment module in response to receiving the activation data.

In another general aspect, a server system includes one or more processing devices and one or more storage devices storing instructions that, when executed by the one or more processing devices, cause the one or more processing devices to receive information indicating a change in an inventory of medical devices, the medical devices in the inventory being maintained in a deactivated state until activated by an enabling device. The instructions cause the one or more processing devices to transmit authorization data to the enabling device associated with the inventory in response to receiving information indicating the change in the inventory. The authorization data is operable to alter a number of medical device activations that the enabling device is permitted to perform. The enabling device is uniquely identified by an identifier for the enabling device or a user of the enabling device. The authorization data is operable to alter the number of medical device activations that the enabling device identified by the identifier is permitted to perform. The authorization data is inoperable to alter the number of medical device activations that other enabling devices are operable to perform.

Implementations may include one or more of the following features. For example, the instructions causing the one or more processing devices to receive information indicating a change in an inventory of medical devices includes the instructions causing the one or more processing devices to receive information indicating an increase in the inventory of medical devices. The instructions causing the one or more processing devices to, in response to receiving information indicating the change in the inventory, transmit authorization data include instructions causing the one or more processing devices to, in response to receiving information indicating the increase in the inventory, transmit authorization data increasing the number of medical device activations that the enabling device is permitted to perform by the amount of the increase in the inventory.

In another general aspect, a medical device includes a treatment module configured to apply a treatment to a patient. The medical device includes one or more data storage devices and one or more processing devices configured to: disallow treatment using the treatment module until activation data is received, receive activation data, and in response to receiving the activation data, permit treatment to be applied using the treatment module.

Implementations may include one or more of the following features. For example, the treatment module includes at least one ultrasound transducer and at least one driver circuit coupled to the ultrasound transducer. The medical device includes a wireless communication module operable to receive the activation data over a wireless communication link. The wireless communication link is a Bluetooth or cellular communication link. The medical device is configured to receive the activation data from an enabling device configured to activate a limited number of medical devices and to not activate more than the limited number of medical devices. The one or more processing devices being configured to receive the activation data includes the one or more processing devices being configured to access the activation data from a removable medium. The activation data includes authorization information indicating a number of treatments authorized using the medical device.

In another general aspect, an enabling device includes an activation module operable to activate a medical device that includes a treatment module having at least one ultrasound transducer and at least one driver circuit coupled to the ultrasound transducer, the medical device being inoperable to apply a treatment using the treatment module until activated by an enabling device. The enabling device includes one or more processing devices and one or more storage devices storing instructions that, when executed by the one or more processing devices, cause the one or more processing devices to: receive information indicating a number of medical device activations that the enabling device is authorized to perform and store, on the one or more storage devices, authorization information indicating the number of medical device activations that the enabling device is currently authorized to perform. The instructions cause the one or more processing devices to receive input from a user indicating that the medical device should be activated, the user being associated with the enabling device, and determine, based on the authorization information, whether activation of the medical device is authorized. If the determination indicates that the activation is authorized, the instructions cause the one or more processing devices to control the activation module to activate the medical device and update the authorization information to decrease the number of activations that the enabling device is authorized to perform. If the determination indicates that the activation is not authorized, the instructions cause the one or more processing devices to control the activation module such that the activation of the medical device is not performed.

Implementations may include one or more of the following features. For example, the user is associated with the enabling device by an identifier for the user. The activation module is operable to activate the medical device by transmitting activation data to the medical device. The activation module is further operable to receive an activation confirmation message indicating that activation was successful. The instructions cause the one or more processing devices to update the authorization information to decrease the number of activations that the enabling device is authorized to perform in response to receiving an activation confirmation message.

Other implementations of these aspects include corresponding computer programs, configured to perform the actions of the methods, encoded on computer storage devices. One or more computer programs can be so configured by virtue having instructions that, when executed by data processing apparatus, cause the apparatus to perform the actions. Other implementations of these aspects also include corresponding computer programs, including the instructions stored on one or more storage devices of the various apparatus and systems, encoded on computer storage devices.

The details of one or more implementations are set forth in the accompanying drawings and the description below. Other features and advantages will become apparent from the description, the drawings, and the claims.

### DESCRIPTION OF DRAWINGS

Fig. 1 is a perspective view of a medical device.
Fig. 2 is a block diagram of the medical device.
Fig. 3 is a diagram illustrating a system for collecting information related to a medical device.
Fig. 4 is a flow diagram illustrating a process for sending information from a medical device.
Fig. 5 is a flow diagram illustrating a process for collecting information.
Fig. 6 is a diagram of a process for storing patient information.
Figs. 7 and 8 are diagrams illustrating a system for activating a medical device.

### DETAILED DESCRIPTION

In some implementations, a medical device can record compliance information that indicates occurrences of treatments performed using the medical device. For example, the compliance information can indicate days and/or times that a patient has performed treatments using the medical device. The compliance information can also indicate a degree to which a patient has complied with a particular treatment regimen. The compliance information can be collected at a server system, and the information can be accessed by multiple parties. The level of access that a party receives can be limited based on the party's relationship to the patient. The server system can receive, store, and provide access to compliance information from multiple medical devices operated by different patients. The medical device can also record errors in the operation of the medical device, send the errors to a server system, and receive service information to address the errors.

In addition, or as an alternative, to the above-mentioned features, the medical device can be provided in a deactivated state, and treatment using the medical device can be disallowed until the device is activated by an enabling device. The enabling device can transmit an activation code to the medical device over a wireless communication link. In response to the activation code, the medical device permits treatments to be performed. The enabling device can be limited to performing a number of activations of medical devices specified by a server system. The server system can monitors inventory levels for a sales representative associated with the enabling device, and can send authorization codes to the enabling device to adjust the number of medical device activations that the enabling device can perform.

Some implementations of the medical device may provide the following additional advantages. Compliance information may be collected and sent to a server system. Security measures can be implemented so that insurance companies, physicians, and caregivers can receive access to the compliance information based on their relationship to the patient. The server system may receive information about errors that occur during operation of the medical device, and the server system may send service information to address the errors.

Some implementations of the medical device may provide the following additional advantages. Medical devices can be maintained in a deactivated state until activated in a controlled process, reducing incentive for theft and misuse. The ability of activation or enabling devices to activate medical devices or to authorize medical treatments can be controlled. The number of medical device activations that can be performed by a device can be limited to an inventory associated with the device.

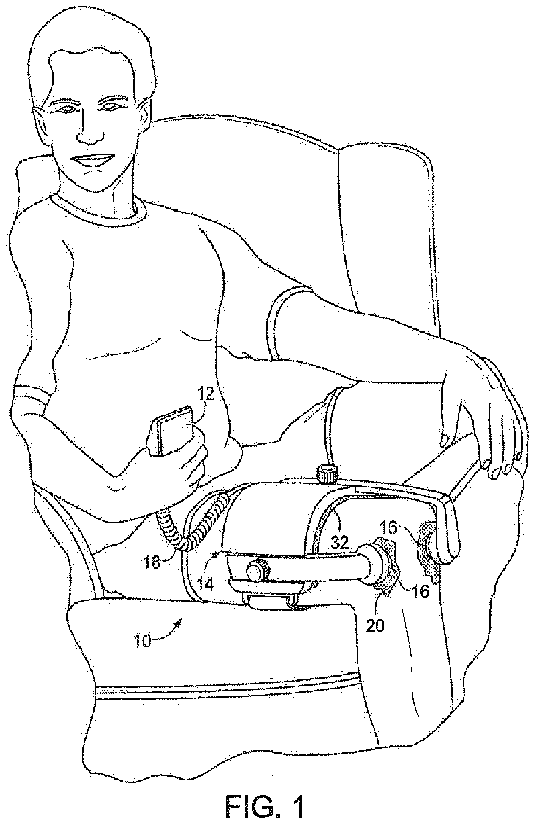

Referring to Fig. 1, a patient is shown using a medical device 10 that includes a treatment module for applying a treatment to the patient. The medical device 10 is a portable ultrasonic treatment device that is equipped to provide purchasable treatments. The treatment module may include, for example, one or more ultrasound transducers 16 and at least one driver circuit coupled to the ultrasound transducers 16.

The medical device 10 can include a control unit 12 that controls the operation of the transducers 16. The control unit 12 can include the transducer driver circuit. The medical device 10 can also include cables 18 that can carry power, data, and control signals between the control unit 12 and the transducers 16.

The medical device 10 can include a placement module 14 that couples the transducers at a location of the patient's body where treatment is needed, for example, over a fractured bone or next to damaged connective tissue. The placement module 14 can include a band, sleeve, applicator, or other connector to fasten the one or more transducers to a treatment site. An ultrasound conducting gel 20 can be applied to the skin of the patient to enable the ultrasound to propagate effectively to the patient's tissue.

The medical device 10 can use low intensity, high-frequency acoustic energy (ultrasound) to treat injuries, defects, or pathologies, For instance, the ultrasonic treatment device can be designed to treat injuries, defects, or pathologies of bones or connective tissue, and, in some instances, can increase cellular level activity that leads to healing of ischaemic or grafted tissue. The medical device 10 may be used as an adjunct to surgical repair, in order to speed healing, or in some cases can be used alone to heal tissue injuries without surgery (e.g., for degenerative diseases such as osteoarthritis, tendonosis, and tendonitis). The medical device 10 can be suitable for use in treatment of bone fractures and/or connective tissues associated with joints, such as those in the hand, foot, wrist, ankle, knee, elbow, hip, shoulder, back, and neck.

For example, following surgery, the medical device 10 can be applied non-invasively to the outside of the body (e.g., coupled to the skin with coupling media, such as a gel) in the region of the repaired tissue. The medical device 10 can be operated to transmit ultrasound (for example, in the form of pulses) into the tissue in need of treatment, or at the interface with the uninjured tissues. Exposure to the ultrasound can stimulate a faster, better quality repair of the tissue. At a bone interface, the ultrasound can also stimulate bone repair and bone ingrowth into repair or graft tissue. This can give rise to a faster, stronger repair and improved integration of the interface between, for example, tendon, ligament, and bone. The ultrasonic treatment device may also be used to non-invasively treat pathologies of connective tissues, such as osteoarthritis, ligament and tendon conditions, without the need for a surgical procedure.

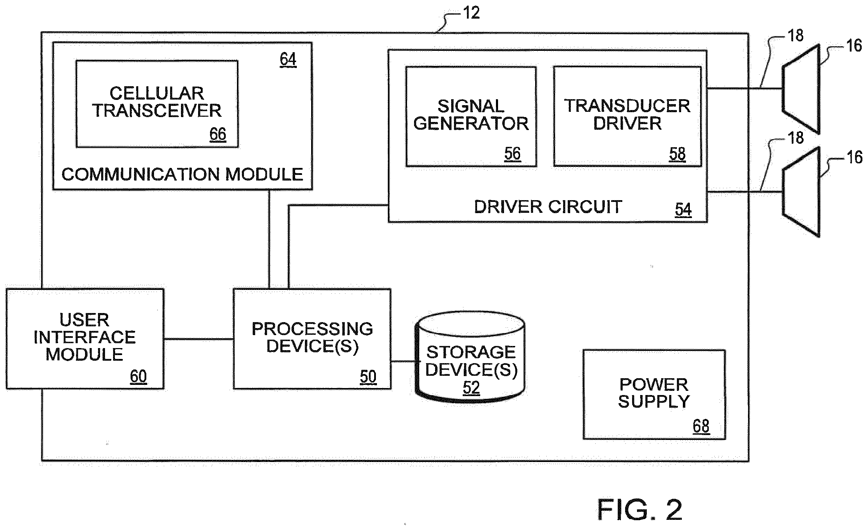

Referring to Fig. 2, the control unit 12 of the medical device 10 can include a processing device 50 that executes instructions stored on a storage device 52. The processing device 50 can include one or more processing devices. The storage device 52 can include one or more storage devices, one or more of which may be removable. The control unit 12 can also include a driver circuit 54, a user interface module 60, a communication module 64, and a power supply 68.

By executing the instructions stored on the storage device 52, the processing device 50 can, for example, control a treatment module (for example, driver circuit 54 and transducers 16) to apply a medical treatment in response to user input. Applying the treatment can include controlling the driver circuit 54 to produce ultrasound with therapeutic properties. Controlling the driver circuit 54 to produce ultrasound can include activating the driver circuit 54, for example, supplying power to the driver circuit 54, sending control signals to the driver circuit 54, or causing the driver circuit 54 to produce a particular output.

If the medical device 10 is in a disabled state, or if treatment with the medical device 10 is not authorized, the processing device 50 can control the treatment module such that the treatment is not applied. For example, the processing device 50 can control the driver circuit 54 such that ultrasound with therapeutic properties is not produced. Controlling the driver circuit to not apply treatment can include not activating the driver circuit 54, deactivating the driver circuit 54, setting the output of the driver circuit 54 (for example, setting the amplitude to zero), and/or otherwise limiting or preventing treatment. The processing device 50 can also be configured to control other components described below, for example using instructions stored on the storage device 52.

The storage device 52 can store a device identifier, such as a serial number, that identifies the particular medical device 10. The device identifier can uniquely identify the medical device 10 and distinguish it from all other ultrasonic treatment devices, even those of the same type or model.

The driver circuit 54 can be configured to send drive signals that cause the transducers 16 to generate ultrasound with therapeutic properties. The driver circuit 54 can include a signal generator 56 that generates a signal and a transducer driver 58 that drives the transducers 16 according to the generated signal. In some implementations, the ultrasound generated by the transducers 16 can include low intensity ultrasound (for example, 100mW/cm² or less) having a frequency ranging between about 1 and 2 MHz, more particularly about 1.5 MHz. The ultrasound can be pulsed, with a pulse width ranging from about 10 to 2,000 microseconds, more particularly about 200 microseconds, with a repetition frequency ranging from about 100Hz to about 10KHz, more particularly about 1 KHz.

The user interface module 60 can provide information to the patient and enable treatment to be initiated. The user interface module 60 may include one or more input devices or controls, for example, buttons, a keypad, or a touch-sensitive screen. The user interface module 60 may be used by a patient or other person, for example, to enter user input that indicates that a treatment should be administered by the medical device. When the processing device 50 determines that treatment is not authorized, the processing device 50 can provide an indication to the patient on the user interface module 60 that more treatments need to be purchased.

The user interface module 60 may also include one or more output devices, for example a screen, a liquid crystal display, or lights. For example, the user interface module 60 can include a screen 72, for example, a liquid crystal display (LCD), a thin-film transistor (TFT) display, a field sequential display, or an organic light-emitting diode (OLED) display. The user interface module 60 can also include light-emitting diodes (LEDs) and other indicators. The user interface module 60 may include a speaker or other device that can produce sound (not shown), or other output devices. The user interface module 60 may also include input capabilities or input devices (not shown), for example, buttons, one or more keypads, and other controls. The screen 72 may be touch-sensitive to receive input from a user. The user interface module 60 can also include an interface to access a removable storage medium, such as a subscriber identity module (SIM) card, a Secure Digital (SD) card, or other types of removable storage media.

The communication module 64 can be configured to send compliance information to a remote system and/or receive information that affects the operation of the medical device 10. In some implementations, the processing device 50 is configured to receive an activation code or other activation data through the communication module 64 and to store the received activation code in the storage device 52. The communication module 64 can enable communication with a server system, client system, or other computer system over a wired or wireless connection. The communication module 64 may enable a communication link that is wired or wireless. The communication module may enable communication over, for example, Ethernet, Universal Serial Bus, 502.11, Bluetooth, Zigbee, cellular networks, and other communication links. In one implementation, the communication module 64 can include a cellular transceiver 66 to receive and/or transmit information over a cellular network. The communication module 64 may also enable communication through multiple communication links.

A power supply 68 can provide power to the components of the medical device 10, including the driver circuit 54, the processing device 50, the storage device 52, the payment module 62, the communication module 64, and the user interface module 60. The power supply 68 can include a battery that is integrated into the control unit 12 or is removable. The battery can be primary battery or a rechargeable battery, and the power supply 68 can include a detachable power adapter that can charge a rechargeable battery.

When a user performs treatment using the medical device 10, the medical device 10 can collect and store compliance information. Collecting compliance information can include recording information about use of the medical device 10, for example, recording information that indicates occurrences of medical treatments using the medical device 10. Compliance information can include a number of treatments provided by the medical device 10, a date and time that a treatment was provided by the medical device 10, and/or a duration that a treatment was provided by the medical device 10. Information about multiple uses or treatments with the medical device 10 can be collected.

A treatment regimen that identifies a prescribed use of the medical device 10 can be identified, and data that indicates the treatment regimen can be stored on the storage device 52. For example, the treatment regimen may be entered on the device after the health condition has been diagnosed or after the medical device 10 has been prescribed to the patient. Information about a treatment regimen may be entered on the medical device 10 or received from a network, which may include a cellular network. The information about the recorded use of the medical device 10 can be compared to the information about the prescribed use of the medical. Information indicating the degree that the recorded use matches the prescribed use can be generated, stored on the storage device 52, and communicated to other devices.

Compliance information can be stored on the storage device 52, on a removable medium, or both the storage device 52 and a separate removable medium. The compliance information may, but is not required to, include one or more results of a comparison between the recorded use of the medical device 10 and scheduled or planned use of the medical device 10, for example, as indicated by treatment regimen of the patient.

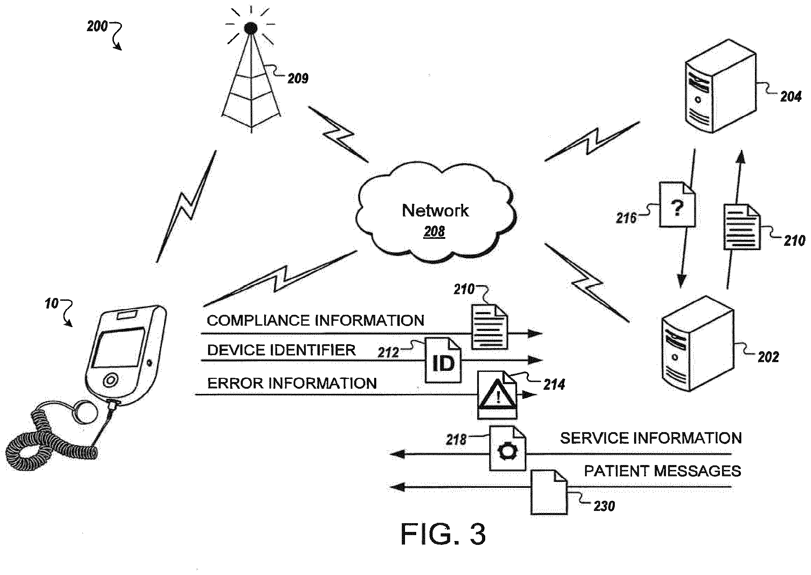

Referring to Fig. 3, an example of a system 200 for collecting compliance information includes the medical device 10, a server system 202, and a third-party system, all connected by a network 208. The medical device 10 and the server system 202 may, additionally or alternatively, communicate through a cellular network 209.

The medical device 10 can record use of the medical device 10, for example, by recording the date, time, and duration that treatment occurs using the medical device 10. The recorded use of the medical device 10 can be collected as compliance information 210. For example, the medical device 10 can collect information about multiple treatments performed or multiple aspects of treatment.

As described above, in some implementations, the medical device 10 may receive information about the treatment regimen for the patient. For example, the treatment regimen may be entered on the device after the health condition has been diagnosed or after the medical device 10 has been prescribed to the patient. The compliance information 210 may, but is not required to, include one or more results of a comparison between the recorded use of the medical device 10 and the treatment regimen of the patient.

To distinguish the medical device 10 from other medical devices 10, the medical device 10 may store a device identifier 212 that enables the medical device 10 to be identified. For example, the device identifier 212 may uniquely identify a particular medical device 10.

The medical device 10 can send the compliance information 210 to the server system 202. Compliance information 210 can be sent automatically, for example, after a predefined number of treatments are performed, after a particular amount of time has elapsed, or after a treatment regimen has been completed. The medical device 10 may send the compliance information 210 over the network 208. Additionally, or alternatively, medical device 10 may send the compliance information 210 over a cellular network 209. The medical device 10 can also send the device identifier 212 to the server system 202 with the compliance information 210, enabling the server system 202 to associate the compliance information 210 with the particular device. In addition to a device identifier, or alternatively, the medical device 10 may send a patient identifier with the compliance information 210.

The server system 202 can be configured to receive the compliance information 210 and the device identifier 212 from the medical device 10. The server system 202 can determine a patient identifier using the device identifier 212. For example, the server system 202 may store records that associate device identifiers 212 for multiple medical devices 10 with patient identifiers. The server system 202 can compare the received device identifier 212 to stored device identifiers to determine a patient identifier and identify the patient that uses the medical device 10. The server system can store the compliance information 210 in association with the determined patient identifier.

The server system 202 can be configured to receive compliance information from multiple medical devices 10 operated by different patients. For example, the server system 202 may include a compliance database of many patients and information about the prescriptions and medical devices 10 that correspond to each patient. The server system 202 can receive and record compliance information from each or any of patients or medical devices 10 described in the compliance database. The server system 202 can add information about additional patients and medical devices 10 to the compliance database records.

The server system 202 can provide access to the stored compliance information 210 to one or more parties that have a relationship with the patient. For example, an insurance company for the patient may use the compliance information 210 to determine whether the patient is using the medical device 10 that the insurance company paid for. A physician or a caregiver for the patient may use compliance information 210 to determine if the patient is complying with a treatment regimen that has been prescribed. To obtain compliance information 210 for the patient, a third-party system 204 may submit an inquiry requesting the compliance information 210 to the server system 202. For example, the inquiry 216 may include one or more patient identifiers to identify one or more patients. The server system 202 can provide access to the compliance information 210 for one or more users in response to receiving the inquiry from the third-party system 204.

Because the server system 202 can store compliance information about multiple patients and multiple medical devices 10, the server system 202 can provide aggregate information about multiple patients and medical devices 10. For example, the server system 202 may provide an insurance provider with a summary of the treatments performed for all patients covered by the insurance provider. As another example, a physician may receive compliance information for each of his patients from the server system 202, without being required to interface with each of the prescribed medical devices 10 individually. Summaries, reports, graphs, and comparisons can be provided based on compliance data for multiple patients, including, for example, information about a set or subset of patients. For example, the compliance of patients that have a particular health condition can be provided.

Access to the compliance information 210 can be limited based on the relationship of the third party to the patient. The server system 202 can store records that associate various third parties with various patients. The third party system 204 may be required to be authenticated or comply with other security measures before access to compliance information 210 is provided. Access to compliance information 210 can also be limited by restricting the quantity or detail of information available. For example, one third party may receive more detailed compliance information 210 than a different third party may receive for the same patient. For example, an insurance company for the patient may be provided access only to the number of treatments performed with the medical device 10, but the physician of the patient may be provided access to the particular dates and times that treatments occurred in addition to the total number of treatments.

The medical device 10 can also detect and record information about errors of the medical device 10. During treatment or during other operation of the medical device 10, one or more errors may occur. The medical device can store information about the errors as error information 214, and can send the error information 214 to the server system 202 with the device identifier 212. In one implementation, error information 214 can be sent soon after the error is detected. Examples of errors that can be detected, and for which information can be recorded and sent, include a gel error indicating that there is insufficient ultrasound conductive gel on a transducer, a battery error that indicates that remaining power of the battery is low, and a connectivity error that indicates that a wire to a transducer is disconnected or broken.

The server system 202 can be configured to receive the error information 214 and the device identifier 212. The server system 202 can store the received error information 214 and can associate error information 214 with the device identifier 212. The received error information 214 may quickly and accurately indicate which medical devices 10 and which types of medical devices 10 experience errors and at what frequency errors occur.

The received error information may also enable the server system 202 to provide information to the medical device 10 to address the errors. For example, the server system 202 may use error information 214 to determine a possible cause of an error. The server system 202 may select service information 218 that addresses the error. For example, the server system 202 may select information to store on the medical device 10, which may include information to restore or replace outdated or incorrect information. The server system 202 may select control instructions to be executed on the medical device 10, for example, control instructions to clear an error or to reinitialize the medical device 10, The server system 202 can send the selected service information 218 that addresses one or more errors to the medical device 10. The service information 218 can include, for example, software or firmware updates, instructions to the user of the medical device 10, instructions to trained service personnel, and/or control instructions to alter the functioning of the medical device 10 and modules coupled to the medical device 10.

The server system 202 can send information including one or more messages 230 to the medical device 10. The messages 230 may be stored on the medical device 10 and displayed to the patient during treatment. The messages 230 can include updated information or additional information to add to messages 230 already stored on the medical device 10. In addition, one or more messages 230 can be provided to instruct the patient how to correct an error of the medical device 10, or to inform the patient that an error has been corrected.

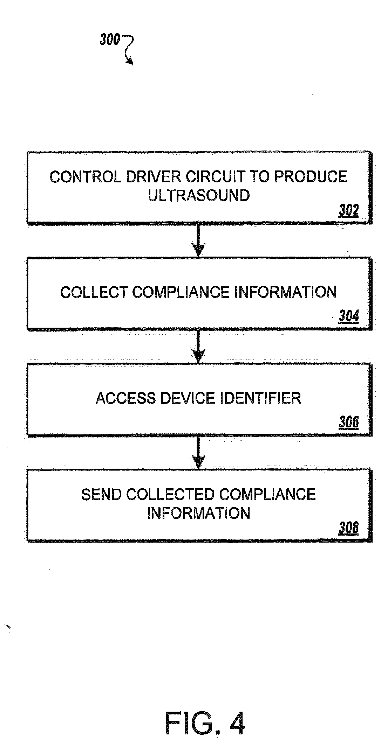

Referring to Fig. 4, a medical device 10 may collect and send information as illustrated in the process 300. The processing device 50 of the medical device 10 may, for example, execute instructions stored on the storage device 52 to perform the process 300.

A driver circuit is controlled to produce ultrasound with therapeutic properties (302).

Compliance information is collected (304). Collecting compliance information can include recording information about use of the medical device, for example recording the number of treatments that are performed. Compliance information can include a number of treatments provided by the medical device, a date and/or time that a treatment was provided by the medical device, and/or a duration that a treatment was provided by the medical device. Information about multiple uses or treatments with the medical device can be collected.

A treatment regimen that identifies a prescribed use of the medical device can be identified. For example, information about a treatment regimen may be entered on the medical device or received from a network, which may include a cellular network. The information about the recorded use of the medical device can be compared to the information about the prescribed use of the medical. Information indicating the degree that the recorded use matches the prescribed use can be generated and stored as compliance information.

A device identifier can be accessed (306). The device identifier can be stored on the medical device. The compliance information can be sent (308). The accessed device identifier can be sent with the compliance information. For example, the compliance information can be sent to a server system configured to receive the compliance information. Compliance information can be sent automatically after a predetermined number of treatments have been performed. Compliance information may be sent using a wireless module, and the wireless module can include a cellular transceiver. For example, compliance information may be sent to a server system over a cellular network using the cellular transceiver. The medical device can include a SIM card that associates a particular telephone number with the medical device.

In some implementations, compliance information can additionally or alternatively be stored on a removable data storage device such as an SD card or other memory card. The removable data storage device can be removed from the medical device 10 and coupled to a computer system. The computer system can then transmit the compliance information (with the associated device identifier) to the server system.

One or more errors of the medical device can be detected. Information about the detected errors can be sent with the accessed device identifier to a server system. Service information to address the detected errors can be received from the server system.

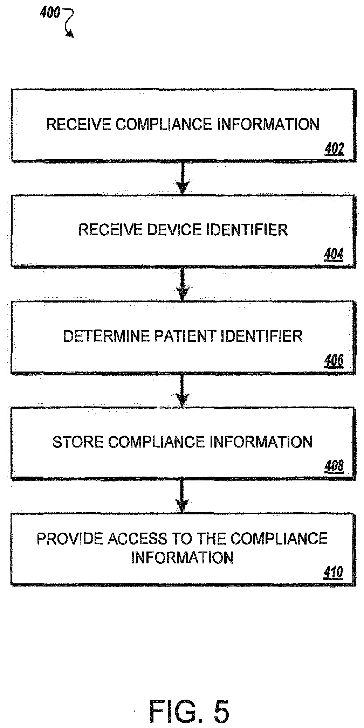

Referring to Fig. 5, a server system can process information according to the illustrated process 400. For example, one or more storage devices can store instructions that, when executed by one or more processing devices, cause the server system to perform the process 400.

Compliance information can be received (402). For example, compliance information can be received over a network or cellular network from one or more medical devices. Compliance information for multiple medical devices operated by different patients can be received. For example, the server system may be configured to receive compliance information from each of a plurality of patients, and each patient can be associated with at least one of a plurality of medical devices. The compliance information can include a number of treatments provided by a medical device, a date and time that a treatment was provided by the medical device, and/or a duration that a treatment was provided by the medical device. Compliance information can be received for multiple medical devices operated by different patients.

A device identifier can be received (404). The device identifier can be received with the compliance information. A patient identifier can be determined (406). For example, the received device identifier can be used to determine the patient identifier. The server system can store records that associate device identifiers with one or more patient identifiers. The recorded device identifiers can be compared to the received device identifier to determine a recorded device identifier that matches the received device identifier.

The compliance information can be stored (408). The compliance information can be stored in association with the patient identifier that was determined to correspond to the compliance information. For example, the server system may be configured to store compliance information for each of a plurality of patients, and each patient can be associated with at least one of a plurality of medical devices. Compliance information for each patient can be stored in association with one or more device identifiers and/or patient identifiers.

Access to the compliance information can be provided (410). For example, access may be provided to one or more users of the server system. Users may submit an inquiry to the server system, and the inquiry can be associated with a patient identifier. Users can include, for example, one or more of a representative of an insurance provider of the patient, a physician of the patient, and a caregiver for the patient. Access to the compliance information for a particular patient can be provided to the users in response to receiving the inquiry that is associated with the patient identifier for the particular patient. Access to the compliance information can be limited based on the relationship of the user to the patient. For example, a physician of the patient may receive access to only a portion of the compliance information, such as only the number of times the medical device was used. A physician, on the other hand, may receive access to more detailed compliance information, such as the date, time, and duration that the medical device was used.

Because compliance information for multiple patients and multiple medical devices can be stored, access to compliance information for multiple patients and multiple medical devices can be provided. For example, compliance information can be provided to a third party for treatment performed by multiple medical devices operated by multiple patients. Users that can receive access to the compliance information can include, for example, one or more caregivers, physicians, and representatives of insurance providers for any of the multiple patients whose compliance information is stored. Access can be provided to compliance information for each of a plurality of patients, where each patient is associated with at least one of a plurality of medical devices. Access to the compliance information for a particular patient can be provided to the users in response to receiving the inquiry that is associated with the patient identifier for the particular patient.

Information about one or more errors of the medical device can be received. Based on the information about the errors, service information to address the errors can be selected and transmitted to the medical device. One or more messages for the patient can be transmitted to the medical device.

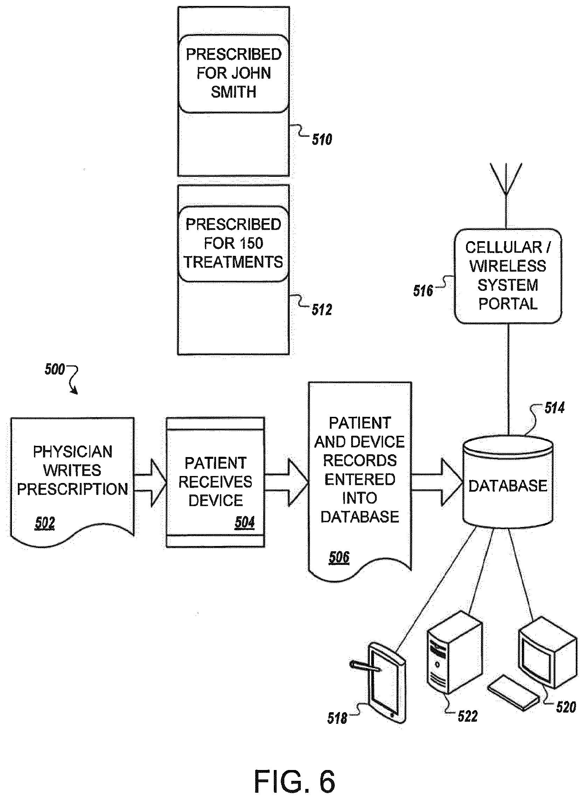

Referring to Fig. 6, a process 500 for storing patient information can begin with a physician writing a prescription for a patient for treatment using a medical device (502). To carry out the prescribed treatment, a medical device can be dispensed to the patient (504). The medical device can be authorized at the time the medical device is dispensed or at a later time.

Records for the patient and the dispensed medical device can be entered into a database 514 (506). For example, the database 514 may store a patient record 510 that associates a particular medical device or treatment with a particular patient, in the example, a patient named "John Smith." The database 514 may also store a prescription record 512 that indicates the number of treatments that can be purchased for the patient. The number of treatments indicated in a prescription record 512 may be authorized for application by the medical device after payment has been received for the prescribed treatments. For example, the treatments can be enabled after a third-party payer agrees to pay for the treatments or after the patient enters payment at the medical device. The database 514 may also store other records including records that identify patient identifiers and medical device identifiers.

The information in the database can be accessed by one or more client devices 518, 520, a server system 522, or other systems. For example, the server system 522 may use the patient record 510 to match payment to a particular medical device or patient. The records stored in the database 514 may also be used to inform a third-party payer or patient of the number of treatments that should be purchased to enable a treatment plan to be carried out.

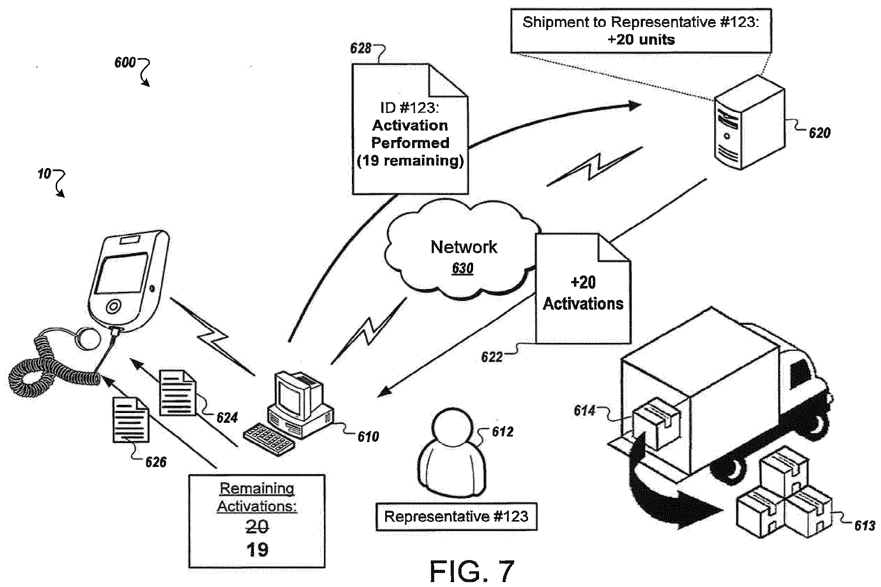

Referring to Fig. 7, an inventory tracking and medical device activation system 600 can be used to control the distribution of medical devices 10. The system 600 includes an enabling device 610 that communicates with the medical device 10. The enabling device 610 also communicates with a server 620 over a network 630.

Each medical device 10 can be provided to a distributor or physician in a disabled or deactivated state. Because the medical devices 10 are shipped and stored in inventory in an inoperative state, the potential for unauthorized use is very low. For example, the medical device 10 can be provided in a state in which no treatments are authorized. In addition, or as an alternative, the medical device 10 can be provided in a state in which treatments cannot be purchased or authorized, until the medical device 10 is activated and thus made operative by an enabling device. Activation of a medical device can be an event that occurs only once, before an initial use of the medical device. In some implementations, the medical device 10 can be pre-authorized to perform medical treatments, but may nonetheless be limited from performing the authorized medical treatments until activation occurs. Activation of the medical device 10 can thus be separate from authorization of treatment using the medical device 10, which can occur in response to payment or the issuance of a prescription for use of the medical device 10.

The enabling device 610 includes the capability to activate medical devices 10. For example, the enabling device 610 can include an activation module including a wireless or wired communication system to transmit activation information. The server 620, however, can limit the enabling device 610. For example, the server 620 can authorize the enabling device 610 to activate only a limited number of medical devices 10. The total number of medical device activations that the enabling device can be performed can be limited (e.g., no more than 10 activations, until further authorization is received). Additionally, or alternatively, the number of activations that can be performed over a particular period of time can be limited (e.g., no more than 10 activations per month). As described in further detail below, the server 620 can limit the number of medical device activations that the enabling device 610 can perform so that, at any given time, the enabling device 610 is authorized to perform a number of medical device activations no greater than the number of medical devices in a particular inventory 613 of a particular sales representative 612. The enabling device 610 is associated with the sales representative 612, and a unique identifier for the enabling device 610 or for the representative 612 associates the enabling device 610 with the inventory 613 of the representative 612.

The server 620 tracks the inventories of multiple representatives. The server 620 can identify changes in inventories using information from reliable sources, for example, information that is verifiable or outside the control of the representatives. For example, the server 620 receives information from manufacturers or distributors of medical devices about shipments of products to the representatives.

Based on the inventory 613 for the representative 612, the server 620 adjusts the ability of the enabling device 610 to activate medical devices 10. For example, the server 620 authorizes the enabling device 610 to activate as many medical devices 10 as are in the official inventory 613 for the representative 612. For example, when the medical devices 10 are shipped to the representative 612, the server 620 receives information about the shipment 614 from the manufacturer or distributor. In response to determining that the inventory 613 for the representative 612 has increased, the server 620 transmits to the representative's enabling device 610 an authorization code 622 or other authorization data that permits a number of medical device 10 activations corresponding to the size of the shipment 614. If twenty medical devices 10 are shipped to the representative 612, the server 620 sends an authorization code 622 permitting the enabling device 610 to activate up to twenty medical devices 10.

When the medical device 10 is purchased or dispensed to a patient, the sales representative 612 can activate the medical device 10 using the enabling device 610. The enabling device 610 communicates with the medical device 10 over a wired link or a wireless link, such as Bluetooth. For example, the enabling device 610 supplies an activation code 624 or other activation data to the medical device 10 that unlocks the functionality of the medical device 10. The enabling device 610 can also supply an authorization code 626 that authorizes a particular number of treatments to be performed with the medical device 10. The activation code 624 and the authorization code 626 can be combined in a single message or code. After the medical device 10 receives the activation code 624, the medical device 10 determines whether the activation code 624 is valid to activate the medical device 10 and/or one or more of its treatment modules. In response to determining that the activation code 624 is valid, the medical device 10 may send an activation confirmation message (not shown) to the enabling device 610 to indicate that activation was successful.

After the enabling device 610 activates the medical device 10, the enabling device 610 automatically decreases the number of activations that the enabling device 610 can provide. For example, the number of activations permitted is decreased by one, from twenty to nineteen. The number of activations can be decreased in response to receiving the activation confirmation message from the medical device 10 so that the number is decreased only after successful activation attempts.

Once the activations allowed by the authorization code 622 are exhausted, the enabling device 610 is restricted from activating additional medical devices 10. The representative 612 is thus restricted from activating medical devices 10 beyond those legitimately in the representative's inventory 613. When a new shipment of medical devices 10 is sent to the representative 612, the server 620 transmits a new authorization code that permits the enabling device 610 to activate the medical devices 10 in the new shipment.

In some implementations, the server 620 can also communicate with the enabling device 610 to reduce the number of activations allowed, for example, if the inventory 613 of the representative 612 decreases due to returning unused medical devices to the manufacturer. Accordingly, the number of activations that can be provided by the enabling device 610 is maintained according to the current inventory 613 of the representative 612.

In some implementations, the enabling device 610 sends a message 628 to the server 620 that indicates when an activation of a medical device 10 has occurred, allowing the server 620 to monitor activations. The remaining number of activations currently allowed by the enabling device 610 can be included in the message 628.

In some implementations, after the medical device 10 expends all of the treatments authorized by the authorization code 626, a second authorization code can be received to permit additional treatments to be performed. As described above, the second authorization code can be received in response to payment by a patient or a patient's insurance provider. The second authorization code can be received, for example, over the network 630, from the enabling device 610, or from a removable medium.

By contrast, in some implementations, the medical device 10 returns to an inoperative, deactivated state after the treatments authorized by the authorization code 626 are exhausted. Thus the medical device 10 must be activated with an activation code from the enabling device 610 before further treatments are permitted.

The medical device 10 can be configured to transmit information indicating usage of the medical device 10, including compliance with a treatment regimen, to the enabling device 610 over the same communication link used to receive the activation code. Thus, as part of the exchange, the medical device 10 receives a new activation code from the enabling device 610, and the enabling device 610 receives usage information from the medical device 10. The enabling device 610 can then transmit the usage information to the server 620, with identifiers to identify, for example, the patient, prescription, and medical device 10 associated with the usage information.

In some implementations, rather than being enabled by a transmission of an activation code from an enabling device, a medical device can be activated by a code accessed from a removable medium, such as an SD memory card.

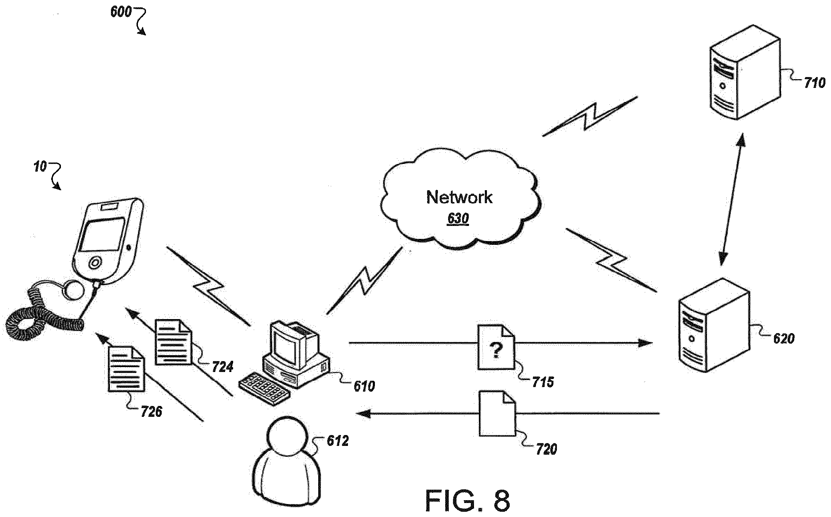

Referring to Fig. 8, the system 600 can also control medical device activations and treatment authorizations based on prescriptions and insurance payment authorizations. For example, the enabling device 610 can be limited to activating a medical device when a valid prescription for the medical device is received, or when an insurance company authorizes payment for a prescribed medical device.

The enabling device 610 can be configured to require an authorization code before performing each activation. Each time a medical device is activated, a new authorization code is required. The server 620 provides authorization codes over the network 630. The server 620 can provide an authorization code allowing the activation of a single medical device based on a particular prescription for the medical device. If no prescription is submitted to the server 620, the server 620 does not send an authorization code to the enabling device 610, and the enabling device 610 is unable to activate medical devices.

For example, after a physician issues a prescription for treatment using the medical device 10, the prescription can be entered on the enabling device 610, which transmits identifying information 715 identifying the prescription to the server 620. For example, the enabling device 610 transmits a representative identifier and a prescription identifier in the identifying information 715. The identifying information 715 may be additionally or alternatively transmitted by the medical device 10, a physician's computer system, an insurance company computer system, or other system. In some implementations, the identifying information 715 can identify the prescription, the patient receiving the prescription, the doctor or office issuing the prescription, an insurance company for the patient, an insurance policy for the patient, the representative 612, the medical device 10 to be activated, and/or the enabling device 610, and the information can be stored by the server 620.

The server 620 verifies the prescription information, for example, by comparing the prescription information to other prescription records or by verifying an authentication signature in the received information. The server 620 can also communicate with another server system 710, such as a server for an insurance company of the patient, to determine whether payment has been authorized for the prescription.

If the prescription is determined to be valid, and/or if payment has been authorized, the server 620 transmits an authorization code 720 to the enabling device 610. The authorization code 720 permits the enabling device 610 to activate a single medical device 10 for the patient and prescription that were verified. The enabling device 610 transmits an activation code 724 to the medical device 10. The enabling device 610 also transmits an authorization code 726 to the medical device 10, permitting the medical device 10 to apply the number of treatments indicated in the prescription, or the number of treatments for which payment was authorized by the insurance company.

The enabling device 610 can transmit information to the server 620 indicating that the activation of the medical device 10 was performed. Based on the initial identifying information 715 and/or information received subsequent to the activation, the server 620 can record the transaction identifying, for example, the patient, the prescription, the medical device 10, the representative 612, the enabling device 610, and the authorization code 720 associated with the activation.

When no prescription is submitted to the server 620, the server 620 does not send an authorization code to the enabling device 610, and the enabling device 610 cannot activate the medical device 10. Similarly, if the prescription information sent to the server 620 is invalid or is not verifiable, or if payment is refused by an insurance provider, the server 620 can withhold the authorization code, disallowing the activation of the medical device 10.

In a similar manner, the system 600 can be used to track and/or evaluate passes for unpaid treatments. In some implementations, the enabling device 610 can be authorized to provide a limited number of complimentary medical device activations or treatment authorizations for previously activated medical devices 10. Each time a pass is provided, the enabling device 610 transmits information to the server 620 identifying, for example, the physician, representative 612, medical device 10, and insurance provider associated with the pass. The information may also identify the patient and prescription associated with the pass. The server 620 can thus maintain a database tracking the issuance of complimentary passes for different representatives 612, physicians, and insurance providers.

In some implementations, the enabling device 610 can be limited to providing passes when the server 620 provides authorization for each pass individually. For example, the server 620 can evaluate the circumstances of a particular patient and a particular prescription to determine whether the patient qualifies for complimentary or reduced cost treatment.

The enabling device 610 transmits a request to the server 620 that indicates the circumstances of the desired pass. The request can indicate, for example, the medical condition to be treated, the identity of the patient, an associated prescription, the patient's income or other financial status, whether and to what degree treatment is covered by insurance, and what insurance provider covers the treatment. The request can also identify the physician, representative 612, enabling device 610, and medical device 10 associated with the request.

The server 620 receives and evaluates the request for the pass. For example, the server 620 uses the information in the request to evaluate the patient's medical needs, economic circumstances, and other circumstances to determine whether to authorize the activation and treatment authorization of a medical device for the patient. If the patient's circumstances meet the criteria for complimentary treatment, the server 620 sends an authorization code to the enabling device 610 permitting the enabling device 610 to provide a pass for the particular patient. If the patient does not qualify for a pass, the server system 620 withholds the authorization code necessary to activate a medical device or authorize additional treatments for the medical device. The server 620 can record each request for a pass, each pass granted, and activations and treatments that occur based on the pass.

The techniques described above are not limited to any particular hardware or software configuration. Rather, they may be implemented using hardware, software, or a combination of both. The methods and processes described may be implemented as computer programs that are executed on programmable computers comprising at least one processor and at least one data storage system. The programs may be implemented in a high-level programming language and may also be implemented in assembly or other lower level languages, if desired.

Any such program will typically be stored on a computer-usable storage medium or device (e.g., CD-ROM, RAM, or magnetic disk). When read into the processor of the computer and executed, the instructions of the program cause the programmable computer to carry out the various operations described above.

A number of implementations have been described. Nevertheless, it will be understood that various modifications may be made. For example, the techniques described can be implemented for medical devices that do not produce ultrasound. A medical device can be activated as described above, and compliance information can be transferred and accessed as described above, for medical devices that include treatment modules other than ultrasound treatment modules.

Accordingly, other implementations are within the scope of the following claims.

## Claims

1. A method of operating a medical device (10), the method comprising:
receiving, via a network (208) or a user input to the medical device, a treatment regimen that identifies a scheduled use of a treatment module associated with the medical device (10), the medical device being configured to communicatively couple to a remote computer system (202);
responsive to receiving an activation code (724), permit medical treatments to be performed by the medical device;
recording information that indicates occurrences of medical treatments using the medical device;
comparing the recorded information to the scheduled use to generate compliance information (210) that reflects a patient's compliance with a treatment regimen involving the treatment module;
accessing from one or more storage devices of the medical device a device identifier (212) that identifies the medical device; and
sending the compliance information (210) with the accessed device identifier (212) to the computer system (202) that is configured to receive the compliance information and the device identifier, determine a patient identifier based on the device identifier, store the compliance information in association with the determined patient identifier, and provide access to the compliance information to one or more users in response to receiving an inquiry associated with the patient identifier.

2. The method of claim 1 wherein the compliance information includes at least one of:
a number of treatments provided by the medical device;
a date or time that a treatment was provided by the medical device; and
a duration that a treatment was provided by the medical device.

3. The method of claim 1 or 2 further comprising:
detecting one or more errors of the medical device; and
sending information about the detected one or more errors with the accessed device identifier to the server computer system.

## Patentansprüche

1. Ein Verfahren zum Betreiben einer medizinischen Vorrichtung (10), wobei das Verfahren Folgendes beinhaltet:
Empfangen, über ein Netzwerk (208) oder eine Benutzereingabe in die medizinische Vorrichtung, eines Behandlungsschemas, das eine geplante Verwendung eines mit der medizinischen Vorrichtung (10) assoziierten Behandlungsmoduls identifiziert, wobei die medizinische Vorrichtung konfiguriert ist, um kommunikativ mit einem entfernten Computersystem (202) gekoppelt zu werden;
als Reaktion auf das Empfangen eines Aktivierungscodes (724), Erlauben, dass medizinische Behandlungen durch die medizinische Vorrichtung durchgeführt werden; Aufzeichnen von Informationen, die auf das Auftreten medizinischer Behandlungen unter Verwendung der medizinischen Vorrichtung hinweisen;
Vergleichen der aufgezeichneten Informationen mit der geplanten Verwendung, um Compliance-Informationen (210) zu generieren, die die Compliance eines Patienten mit einem Behandlungsschema widerspiegeln, an dem das Behandlungsmodul beteiligt ist;
Zugreifen, von einer oder mehreren Speichervorrichtungen der medizinischen Vorrichtung, auf eine Vorrichtungskennung (212), die die medizinische Vorrichtung identifiziert; und
Senden der Compliance-Informationen (210) mit der Vorrichtungskennung (212), auf die zugegriffen wurde, an das Computersystem (202), das zum Empfangen der Compliance-Informationen und der Vorrichtungskennung konfiguriert ist, Bestimmen einer Patientenkennung basierend auf der Vorrichtungskennung, Speichern der Compliance-Informationen in Verbindung mit der bestimmten Patientenkennung, und
Bereitstellen des Zugriffs auf die Compliance-Informationen für einen oder mehrere Benutzer als Reaktion auf das Empfangen einer mit der Patientenkennung assoziierten Anfrage.

2. Verfahren gemäß Anspruch 1, wobei die Compliance-Informationen mindestens eines von Folgenden umfassen:
eine Anzahl von Behandlungen, die durch die medizinische Vorrichtung bereitgestellt wurden;
ein Datum oder eine Uhrzeit, zu dem/der eine Behandlung durch die medizinische Vorrichtung bereitgestellt wurde; und
eine Dauer, während der eine Behandlung durch die medizinische Vorrichtung bereitgestellt wurde.

3. Verfahren gemäß Anspruch 1 oder 2, das ferner Folgendes beinhaltet:
Detektieren eines oder mehrerer Fehler der medizinischen Vorrichtung; und
Senden von Informationen über den einen oder die mehreren detektierten Fehler mit der Vorrichtungskennung, auf die zugegriffen wurde, an das Servercomputersystem.

## Revendications

1. Un procédé de fonctionnement d'un dispositif médical (10), le procédé comprenant :
la réception, par le biais d'un réseau (208) ou d'une entrée d'utilisateur vers le dispositif médical, d'un régime de traitement qui identifie une utilisation programmée d'un module de traitement associé au dispositif médical (10), le dispositif médical étant configuré pour se coupler de façon à communiquer à un système informatique à distance (202) ;
en réponse à la réception d'un code d'activation (724), le fait de permettre à des traitements médicaux d'être effectués par le dispositif médical ;
l'enregistrement d'informations qui indiquent des occurrences de traitements médicaux à l'aide du dispositif médical ;
la comparaison des informations enregistrées à l'utilisation programmée afin de générer des informations d'observance (210) qui reflètent l'observance d'un patient vis-à-vis du régime de traitement impliquant le module de traitement ;
l'accès depuis un ou plusieurs dispositifs de stockage du dispositif médical à un identifiant de dispositif (212) qui identifie le dispositif médical ; et
l'envoi des informations d'observance (210) avec l'identifiant de dispositif ayant fait l'objet d'un accès (212) au système informatique (202) qui est configuré pour recevoir les informations d'observance et l'identifiant de dispositif, déterminer un identifiant de patient sur la base de l'identifiant de dispositif, stocker les informations d'observance en association avec l'identifiant de patient déterminé, et fournir un accès aux informations d'observance à un ou plusieurs utilisateurs en réponse à la réception d'une demande associée à l'identifiant de patient.

2. Le procédé de la revendication 1 dans lequel les informations d'observance incluent au moins un élément parmi :
un nombre de traitements fournis par le dispositif médical ;
une date ou une heure à laquelle un traitement a été fourni par le dispositif médical ; et
une durée pendant laquelle un traitement a été fourni par le dispositif médical.

3. Le procédé de la revendication 1 ou de la revendication 2 comprenant en outre :
la détection d'une ou de plusieurs erreurs du dispositif médical ; et
l'envoi d'informations au sujet des une ou plusieurs erreurs détectées avec l'identifiant de dispositif ayant fait l'objet d'un accès au système informatique serveur.
